# EUROPEAN PATENT SPECIFICATION

(11) **EP 4 624 902 B1**
(45) Date of publication and mention of the grant of the patent: **07.10.2026**
(21) Application number: 24167043.9
(22) Date of filing: 27.03.2024
(51) Int. Cl.: G01N 23/223, G01N 21/31, G01N 21/33, G01N 21/3577, G01N 21/41, G01N 21/359, G01N 21/65, G01N 21/85

(54) **METHOD FOR IN-LINE MONITORING THE CONCENTRATION OF METAL SULFATES, IN-LINE MEASURING SYSTEM AND INSTALLATION ASSEMBLEY**
VERFAHREN ZUR INLINE-ÜBERWACHUNG DER KONZENTRATION VON METALLSULFATEN, INLINE-MESSSYSTEM UND ANLAGENANORDNUNG
PROCÉDÉ DE SURVEILLANCE EN LIGNE DE LA CONCENTRATION DE SULFATES MÉTALLIQUES, SYSTÈME DE MESURE EN LIGNE ET ENSEMBLE D'INSTALLATION

(43) Date of publication of application: 01.10.2025
(73) Proprietor: Endress+Hauser Group Services AG, 4153 Reinach (CH)
(72) Inventor: Rönnbäck, Hans, 19468 Upplands Väsby (SE)
(74) Representative: Endress + Hauser Group Services (Deutschland) AG+Co. KG

(56) References cited:
- WO-A1-2015/093578
- CN-A- 108 529 688
- CN-A- 112 510 193
- CN-A- 113 526 569
- CN-A- 113 860 321
- CN-A- 117 169 263
- KR-A- 20240 000 991

## Description

The invention relates to a method for in-line monitoring the concentration of individual metal sulfates, namely at least Nickel Sulfate (NiSO4), Cobalt Sulfate (CoSO4) and Manganese Sulfate (MnSO4), in a solution comprising at least one metal sulfate. The solution is contained in a pipe or a tank of an industrial plant. The invention further relates to an in-line measuring system, an installation assembly, as well as the use of the installation assembly.

The quality and/or efficiency of active cathode material production (CAM) for so-called NMC based - NMC: short for Nickel (Ni) Manganese (Mn) and Cobalt (Co) -cathode materials depends strongly on a sufficiently precise control of the composition, i.e. the concentration of the individual metal sulfates, in the feedstock preparation. NMC based materials for the positively charged cathode are commonly used in the production of lithium-ion batteries, which are, for example, used in mobile devices and/or electric vehicles. In the above-mentioned feedstock preparation process, aqueous solutions of a mixtures of at least three metals sulfates, namely NiSO4, CoSO4 and MnSO4, as well as potentially additional metal sulfates, are used. The blending of the metal sulfates relies on ratio-feedforward control, which is achieved using flow meters, for example Coriolis-type Mass flow meters, in combination with frequent manually performed grab sample, which are subsequently analyzed by means of a laboratory-type analysis, to properly adjust the measured concentrations of the metal sulfate concentrations to reach pre-set target values. Such manually performed grab samples are time consuming and costly, and eventually hinder the scaling-up of the production process. Additionally, potential influences from residuals and/or impurities from upstream process steps may have an impact for feedforward ratio control of the raw materials.

Therefore, for fast and reliable process control, there is a strong need for a robust method and measuring systems which can in-line perform recurring measurements, for in-line determination and control of the concentration of individual metal sulfates. In the context of this application, "in-line measurement" relates to a measurement which is performed on-site, with measurement instruments fixedly, especially permanently, installed in the process plant, for example in a flow-through or in a bypass of a pipe of an industrial plant. Preferably such in-line measurements may be performed recurringly, especially without interrupting the production process. An in-line measurement refers therefore to the measurement of a measurand directly and immediately on site, without, for example, any further pre-treatments of a sample of the solution being necessary. In contrast, non-in-line measurements which require such pre-treatments lead to a time delay. This means that process tracking based on a measurand not measured in-line makes it difficult to control and/or regulate a process based on measurands not measured in-line.

Relevant prior art is represented by CN 112 510 193 A, WO 2015/093578 A1, KR 2024 0000991 A, CN 113 860 321 A, CN 108 529 688 A, CN 113 526 569 A and CN 117 169 263 A.

It is therefore the object of the invention to provide a robust yet accurate in-line monitoring of the concentration of individual metal sulfates in a solution of several metal sulfates.

The object is achieved by a method for in-line monitoring the concentration of individual metal sulfates, namely at least NiSO4, CoSO4 and MnSO4, in a solution comprising at least one metal sulfate as claimed in claim 1, an in-line Measuring system as claimed in claim 10, an installation assembly as claimed in claim 11, as well as the use of the installation assembly as claimed in claim 15.

Advantageous embodiments are specified in the dependent claims, where the respective embodiments of the method are mutatis mutandis comprised by the measuring system the installation assembly, as well as the use, and vice versa.

Regarding the method, the object is achieved by a method for in-line monitoring the concentration of at least NiSO4, CoSO4 and MnSO4 in a solution comprising at least one metal sulfate, wherein the solution is contained in a pipe or tank of an industrial plant, the method comprising:
- In-line obtaining a UV-VIS measuring spectrum of the solution, especially by using ultraviolet or ultraviolet-visible or near infrared electromagnetic waves having a wavelength within a range from 200 nm to 1000 nm, preferably 300 nm to 900 nm,
- In-line determining the concentration of CoSO4 as a first metal sulfate contained in the solution and the concentration of NiSO4 as a second metal sulfate contained in the solution, based at least on an in-line analysis of the UV-VIS measuring spectrum,
- In-line determining the total metal sulfate concentration contained in the solution, by using at least one in-line measurement which is sensitive to the total concentration of the metal sulfates, wherein the in-line determination of the total metal sulfate concentration is performed in addition to the in-line obtaining of the UV-VIS measuring spectrum, by using a method different from UV-VIS;
- In-line determining the concentration of MnSO4 as a third metal sulfate contained in the solution, based at least on the determined concentration of NiSO4, the determined concentration of CoSO4, and the determined total metal sulfate concentration.

The UV-VIS measuring spectrum is obtained by in-line subjecting the solution to ultraviolet or ultraviolet-visible or near-infrared absorption and/or reflectance spectroscopy or spectrophotometry. This is used to determine the concentration of CoSO4 as a first metal sulfate and NiSO4 as a second metal sulfate.

The at least one in-line measurement for determining the total metal sulfate concentration is sensitive to and therefore indicative of the total metal sulfate concentration.

Depending on the specific application, the individual and total metal sulfate concentrations may be determined in terms of a volumetric concentration, molar concentration, or mass density concentration with the method according to the invention.

Investigations of the applicant have demonstrated that it is feasible to use in-line UV-VIS spectroscopy or spectrophotometry, for a reliable determination the induvial concentration of NiSO4 and CoSO4 in a solution containing one or more metal sulfates. The total metal sulfate concentration, i.e., the total concentration of all metal sulfates contained in the solution, is then used to indirectly determine the concentration of the third metal sulfate, namely MnSO4, which does not have a strong, identifiable absorption/reflectance signature in the UV-VIS measuring spectrum.

In the simplest case, wherein the solution comprises only NiSO4, CoSO4 and MnSO4 as metal sulfates, the MnSO4 concentration may then be determined by subtracting the NiSO4 and CoSO4 concentrations from the determined total metal sulfate concentration.

In an embodiment of the method, wherein in the industrial plant, MnSO4 is admixed to a first solution comprising NiSO4 and CoSO4 as metal sulfates, yielding a second solution,
the determining of the total mass metal sulfate concentration is carried out both with the first solution and with the second solution,
and wherein the determining of the concentration of MnSO4 in the second solution is based at least on:
   - the determined concentration of NiSO4 of the first solution and/or the determined concentration of NiSO4 of the second solution,
   - the determined concentration of CoSO4 of the first solution and/or the determined concentration of CoSO4 of the second solution, and
   - the determined total metal sulfate concentration of the first solution and the determined total metal sulfate concentration of the second solution.

This means, that the determination of the concentration of MnSO4 is based both on measurements performed before and after admixing MnSO4 to the solution.

Additionally, the measurements may also be performed before and after admixing one of the other, preferably before and after admixing each of the other metal sulfates, for a more precise determination of the total and/or individual metal sulfate concentration.

In an embodiment of the method, the at least one in-line measurement for determining the total metal sulfate concentration comprises at least one of the following:
- Raman spectroscopy carried out with an in-line Raman spectrometer, wherein a Raman measuring spectrum is in-line analyzed to determine the total concentration of all metal sulfates in the solution,
- A total mass density measurement carried out with an in-line mass density measuring apparatus,
- A Refractive index measurement carried out with an in-line refractometer.

Only a single one of the above in-line measurement (Raman Spectroscopy, mass density and/or Refractive index) may be used in case the solution is essentially composed of metal sulfates, wherein no significant amount of other types of molecules different from the metal sulfates and contributing to the Raman Spectrum, Mass Density, Refractive index, respectively, are present in the solution. In other cases, the determining the total metal sulfate concentration may comprise more than one of the above measurements and, potentially, the consideration of further in-line measurable process variables.

The in-line mass density measuring apparatus is, for example, embodied as a mass flow meter, especially as Coriolis mass flow meter. The in-line mass density measuring apparatus may, for example, comprise a tuning fork as a mechanically oscillatable unit and/or an U-shaped oscillating tube for guiding the solution, and may be based on a so-called MEMS sensor, The in-line mass density measuring apparatus may also be embodied as an in-line ultrasonic mass concentration meter.

In an embodiment of the method, the solution contains a fourth metal sulfate and the atomic number of the metal of the fourth metal sulfate differs by at least 5, especially at least by 10 from the atomic numbers of the metals of the first, second and third metal sulfates, namely Co, Ni and Mn, the method comprises:
- Performing in-line Gamma-ray or X-ray absorption measurements, especially using a low energy gamma or X-ray source, having energies below 350 keV, especially below 200 keV, and obtaining a Gamma-ray or X-ray measuring spectrum,
- In-line determining the concentration of the fourth metal sulfate, by analyzing the Gamma-ray or X-ray measuring spectrum, and
- In-line determining the concentration of MnSO4 as a third metal sulfate contained in the solution, based at least on the determined concentration of NiSO4, the determined concentration of CoSO4, the determined concentration of the fourth metal sulfate, and the determined total metal sulfate concentration.

In an embodiment of the method, in case the solution contains less than 500ppm, especially less than 100 ppm, of other ions which are no metal sulfates, in relation to the total ion concentration, the at least one measurement method for determining the total metal sulfate concentration an in-line conductivity measurement, carried out with an in-line conductivity sensor.

In an embodiment of the method, the method comprises:
- In-line determining the concentration of H2O2 in the solution, by in-line analyzing the UV-VIS measuring spectrum and/or by in-line analyzing the oxidation reduction potential measured in-line with an in-line oxidation reduction potential sensor, and/or
- In-line determining the concentration of H2SO4, by in-line measuring the pH- value, and
- Considering the determined H2O2 concentration and/or the determined H2O4 concentration in the solution, in the determining of the total and/or individual metal sulfate concentration of the first, second, third, and, if applicable, fourth metal sulfate.

Acid residuals such as H2SO4 and/or H2O2 may influence the determined concentrations mentioned above, especially the ones determined indirectly such as the MnSO4 concentration. Therefore, in this embodiment, a cross-sensitivity of the determined concentrations on the acid residuals present in the solution is advantageously considered.

In an embodiment of the method, the method comprises:
- In-line determining the temperature of the solution, and
- Considering the determined temperature, in the determining of the total and/or individual metal sulfate concentration of the first, second, third, and, if applicable, fourth metal sulfate.

Therefore, in this embodiment, a cross-sensitivity of the determined concentrations on the temperature of the solution is advantageously considered. The determined temperature may also be used to convert volumetric concentrations into mass concentrations, or vice versa.

In an embodiment of the method, the in-line determination of the concentration of the first, the second, the third, and, if applicable, the fourth metal sulfate comprises:
In-line performing a, especially multivariate, analysis, wherein the analysis includes the processing of at least two of the following:
- the UV-VIS measuring spectrum, the Raman measuring spectrum, the determined total mass density, the Gamma-ray or X-ray measuring spectrum, the determined pH-value, the determined conductivity, the determined oxidation reduction potential, the determined concentration of H2SO4, the determined concentration of H2O2, the determined temperature.

The, especially multivariate, analysis may be based on an analytical model, to map the aforementioned measuring spectrum/spectra and/or the aforementioned determined measured values to the concentrations of the metal sulfates to be determined in-line. Of course, more advanced models could be used for such a mapping, for example based on a machine learning or Al model, such as an artificial neural network. Such an artificial neural network is, for example, one of the following:
- a fully connected neural network,
- a single- or multilayer perceptron,
- a multilayer neural network with a large number of intermediate layers,
- a convolutional neural network,
- a recurrent neural network.

In an embodiment of the method, a model used in the, especially multivariate, analysis is calibrated, verified adjusted and/or trained, by using a laboratory spectrometer, especially an inductively coupled plasma atomic emission spectroscopy spectrometer, in a laboratory located in the industrial plant or remote from the industrial plant.

Such laboratory spectrometers, which cannot be used as part of an in-line measuring system, may be advantageously used within the method according to invention, to properly calibrate, verify, adjust the model, and/or, in case of a machine learning or Al model, for the training of said model.

Using a laboratory spectrometer as a reference method, reference measured values for metal sulfate concentrations are acquired. These reference values are mapped to the concentrations determined by the method according to the invention by use of the model, with an acceptable correlation accuracy. This is for example carried out before the in-line determining of the individual metal sulfate concentrations, as part of an initialization process and/or repeated after a set operation time of the in-line measuring system.

The model acquired within said initialization process is then used subsequently in the method, so that the individual metal sulfate concentrations can be determined both in-line and with a sufficient accuracy required in the CAM production process.

Regarding the measuring system, the object of the invention is achieved by an In-line measuring system, wherein the in-line measuring system is embodied to carry out the method according to the invention, wherein the measuring systems comprises:
- an in-line ultraviolet or ultraviolet-visible absorption and/or reflectance spectrometer or spectrophotometer, for in-line determining an ultraviolet-visible or ultraviolet absorption and/or reflectance spectrum,
- at least on in-line measuring apparatus different from the ultraviolet or ultraviolet-visible absorption and/or reflectance spectrometer or spectrophotometer, wherein the in-line measuring apparatus is embodied to in-line determine the total metal sulfate concentration contained in the solution, by using at least one measurement method which is sensitive to the total concentration of the metal sulfates, and
- an evaluation and/or computation unit, embodied to determine the concentration of at least NiSO4, CoSO4 and MnSO4 in the solution.

For example, the system comprises an in-line Raman spectrometer. The in-line Raman spectrometer is used to acquire the in-line Raman measuring spectrum.

As set out below, other embodiments of the measuring systems may comprise additional measuring apparatuses, for example to in-line determine one or more of the aforementioned measuring spectra (Gamma-ray or X-ray measuring spectrum) and/or to in-line determine measurands for measured variables such as the aforementioned total mass density, the pH-value, the conductivity, the oxidation reduction potential, the concentration of H2SO4, the concentration of H2O2, the temperature etc. Additional measured variables may comprise but are not limited to conductivity, viscosity, mass flow rate. For example, with a Coriolis-type mass flow meter, the mass density, viscosity, and mass flow rate are advantageously determined simultaneously.

Regarding the installation assembly, the object of the invention is achieved by an Installation assembly for producing a solution containing metal sulfates in an industrial plant. The installation assembly comprises:
- a tank for containing the solution,
- a pipe system comprising pipes for guiding the solution, wherein the pipes lead into or out of the tank, and
- at least a first in-line measuring system according to the invention, wherein the first in-line measuring system is installed in the pipe system.

In an embodiment of the installation assembly, it comprises:
- a first, second, third, and, if applicable, fourth supply storage for the first, second, third, and, if applicable, fourth metal sulfate, respectively,
- supply pipes leading from the first, second, third, and, if applicable, fourth supply storage to the tank, for producing the solution,

wherein the installation assembly is designed in such a way that the concentration of the first, second, third, and if applicable, fourth metal sulfate in the solution is determined with at least the first In-line Measuring system, on its way from its respective supply storage into the tank, during the admixing of the respective metal sulfate to the solution,
and wherein especially the installation assembly is embodied to in-line blend the first, second, third, and, if applicable, fourth metal sulfate with one another, on their way from their respective supply storage into the tank, preferably using static mixing.

In another embodiment of the installation assembly, it comprises:
a second in-line measuring system according to the invention, wherein the second in-line measuring system is arranged in a circuit pipe, wherein the circuit pipe is leading from a first outlet leading out of the tank to an inlet leading into of the tank
   and/or
comprising a third in-line measuring system according to the invention, wherein the third in-line measuring system is arranged with respect to a feeding pipe, which feeding pipe leads from a second outlet of the tank to a crystallizer in such a way that the concentration of the first, second, third, and if applicable, fourth metal sulfate in the solution is determined on its way from the tank to the crystallizer.

In an embodiment of the installation assembly, the installation assembly is embodied to control the inflow from the supply pipes into the tank, based on the concentration of the first metal sulfate, the second metal sulfate, the third metal sulfate, and/or, if applicable, the fourth metal sulfate, determined in-line with the first in-line measuring system, the second in-line measuring system and/or the third in-line measuring system.

For example, the first, second and/or third in-line measuring system is/are installed in a respective pipe measuring section, which pipe measuring section has a length of less than 3 m in a longitudinal direction of the pipe.

The invention also relates to the use of the installation assembly according to the invention, in a feeding pipe for a crystallizer for crystallization of cathode materials for lithium-ion batteries.

The invention is explained in more detail with reference to the following figures, which are not to scale, where identical reference signs denote identical features. Where clarity so requires or it otherwise appears expedient, reference signs already mentioned are omitted in subsequent figures.

All embodiments of the method are mutatis mutandis comprised by the measuring system as well as the installation assembly, and vice versa.

The following are shown:

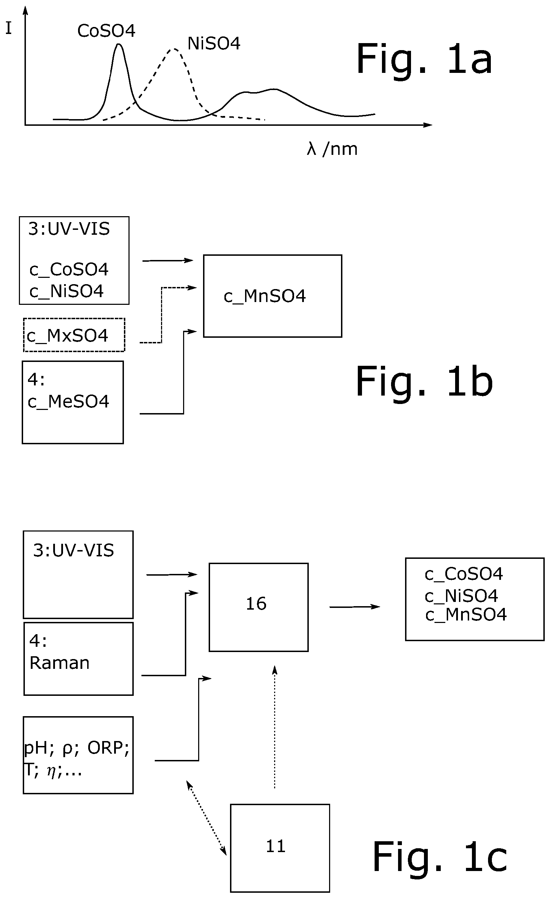

Fig. 1a: A UV-VIS measuring spectrum of a solution containing metal sulfates;
Fig. 1 b: A flowchart of an embodiment of the method according to the invention;
Fig. 1c: A flowchart of an embodiment of the method according to the invention;
Fig. 2 a, b: A schematic view of an embodiment of the in-line Measuring system 100 according to the invention; and
Fig. 3 a, b, c: A schematic view of embodiments of the installation assembly 200 according to the invention.

Fig. 1a shows investigations of the applicant, wherein a first UV-VIS spectrum (Intensity over wavelength) of a solution containing Cobalt sulfate CoSO4 (first metal sulfate, solid line) and a second UV-VIS spectrum of a solution containing Nickel sulfate NiSO4 (second metal sulfate, dashed line) was collected. The investigations demonstrate that the first and second metal sulfate exhibit two clearly distinguishable peaks in the UV-VIS measuring spectrum, comprising a first peak (solid line) for CoSO4 and second peak for NiSO4 (dashed line).

The method according to the invention makes use of this by using an in-line UV-VIS spectrometer or spectrophotometer 3, to in-line determine the concentration of the first and second metal sulfate in a solution 1; 1a; 1b contained in a pipe 2a;2b;2c or a tank 2 of an industrial plant.

Fig. 1b shows a flowchart of steps comprised by the method, in first embodiment of the method according to the invention. First, the cobalt sulfate concentration c_CoSO4 and the nickel sulfate concentration c_NiSO4 contained in the solution 1; 1a; 1b are determined, by in-line obtaining and analyzing the UV-VIS measuring spectrum, especially by using ultraviolet or ultraviolet-visible or near infrared electromagnetic waves having a wavelength within a range from 200 to 1000 nm.

This information is then combined with another in-line measurement, different to the UV-VIS, to determine the total metal sulfate concentration c_MeSO4. This is done in the embodiment of the method shown in Fig. 1b, c by using in-line Raman spectroscopy carried out with an in-line Raman spectrometer 4, wherein a Raman measuring spectrum is in-line analyzed. Without limiting the generality, the invention is explained now and hereinafter in connection with an in-line Raman spectrometer 4 for the determination of the total metal sulfate concentration. The invention is however not limited to using Raman spectroscopy and may, alternatively or additionally, comprise one of the following to determine the total metal sulfate concentration c_MeSO4:
- A total mass density measurement carried out with an in-line mass density measuring apparatus 5,
- A Refractive index measurement carried out with an in-line refractometer 6.
- A conductivity measurement carried out with a conductivity sensor 7.

Once the total metal sulfate concentration c_MeSO4, the cobalt sulfate concentration c_CoSO4 and the nickel sulfate concentration c_NiSO4 are determined, the manganese sulfate concentration c_MnSO4 can then be determined indirectly from the other individual and the total metal sulfate concentrations. For example, wherein the solution comprises only NiSO4, CoSO4 and MnSO4 as metal sulfates, the manganese sulfate concentration c_MnSO4 may be determined by simply subtracting the nickel sulfate concentration c_NiSO4 as well as the cobalt sulfate concentration c_CoSO4 from the determined total metal sulfate concentration c_MeSO4.

Preferably, the individual and/or total metal sulfate concentrations c_NiSO4; c_CoSO4; c_MnSO4; c_MeSO4 are determined at least twice: once for a first solution 1a and once for a second solution 1b, wherein the second solution 1b is obtained by admixing manganese sulfate to the first solution 1a. Thus, total metal sulfate concentration c_MeSO4 of both the first solution 1a and the second solution, before and after admixing the manganese sulfate is determined, for example by using the same Raman spectrometer 4 or several Raman spectrometers 4. This increases the accuracy of the method according to the invention.

Of course, the total metal sulfate concentration c_MeSO4 may also be determined both before and after adding NiSO4 and/or CoSO4, to increase the accuracy of the method according to the invention. Likewise, the accuracy may be increased, when several UV-VIS measuring spectra are obtained and analyzed and the cobalt sulfate concentration c_CoSO4 and nickel sulfate concentration c_NiSO4 are determined both before and after admixing several individual metal sulfates to the solution 1; 1a; 1b.

Additionally (see the optional dashed box in Fig. 1b), in case a fourth metal sulfate is present in the solution 1; 1a; 1b, and the atomic number of the metal of the fourth metal sulfate differs by at least 5, especially at least by 10 from the atomic numbers of the metals of the first, second and third metal sulfate, the concentration of that fourth metal sulfate MxSO4 is additionally determined by using in-line Gammy-ray or X-ray absorption measurements.

Preferably, see Fig. 1c, the UV-VIS measuring spectrum, the Gammy-ray or X-ray measuring spectrum (if applicable) and the one or more measurements to determine the total metal sulfate concentration c_MeSO4 are analyzed by considering one or more additional measurands determined in-line. The additional measurands collected in-line, may comprise measurands one of the following measured variables:
- the pH-value, the conductivity, the oxidation reduction potential, the temperature, the viscosity, etc.

Depending on the presence of ions different from the metal sulfates in the solution 1; 1a; 1b, the in-line conductivity measurement may be itself indicative of the total metal sulfate concentration c_MeSO4, or, too, serve as an additional measured variable considered
to increase the accuracy of the method according to the invention.

For example, acid residuals, such as H2SO4 and or peroxide H2O2 and other impurities may be present in the solution 1; 1a; 1b, especially from upstream process steps. Determining their amount, for example by one or more of the above-mentioned measured variables, also increases the accuracy of the method according to the invention.

The temperature measurement is advantageously used for temperature compensation, for example, when the measured temperature is not stable, as some of the aforementioned physical and/or electrochemical measured variables may exhibit a strong temperature dependence.

In the embodiment shown in Fig. 1c, a model 16 is used to determine the metal sulfate concentrations c_CoSO4, c_NiSO4, c_MnSO4 from the UV-VIS measuring spectrum, the Raman measuring spectrum and the additional measured variables. The term "model" may describe an analytical and/or chemometric model, but also refers to models based on machine learning or Artificial Intelligence (Al).

To validate the model 16 used in the embodiment of the method shown in Fig. 1c, an additional laboratory spectrometer 11 is used to calibrate, verify adjust and/or train the model, preferably at least in an initialization process. The laboratory spectrometer 11 uses laboratory methods and - unlike the UV-VIS spectrometer - cannot be used as an in-line spectrometer. For example, by performing elemental analysis with an elemental laboratory spectrometer 11, the elemental metal concentrations are reliably determined with high accuracy. The determined elemental metal concentrations are then correlated with the metal sulfate concentrations c_CoSO4, c_NiSO4, c_MnSO4 determined with the in-line method according to the invention, in order to calibrate, verify, adjust and/or train the model in said correlation process, in order to achieve a sufficient accuracy for method according to the invention.

The laboratory spectrometer 11 is embodied, for example as an inductively coupled plasma atomic emission spectroscopy (ICP-OES) spectrometer, or an X-Ray Fluorescence (XRF) spectrometer.

The in-line determined metal sulfate concentrations c_CoSO4, c_NiSO4, c_MnSO4,... with the method according to the invention may be advantageously used for an in-line monitoring of the feedstock preparation process for the CAM production. Preferably, recurrent in-line measurements are performed often enough to closely monitor and/or control the process. For example, at least 1 measurement value per 30 minutes, preferably at least 1 measurement value per 10 minutes, especially at least 1 measurement value per 5 minutes is collected with the in-line measurement system 100 (see below), preferably in a periodic manner. At an optimum, an essentially continuous in-line measurement with a measurement frequency of 1 measurement per 1 minute is performed.

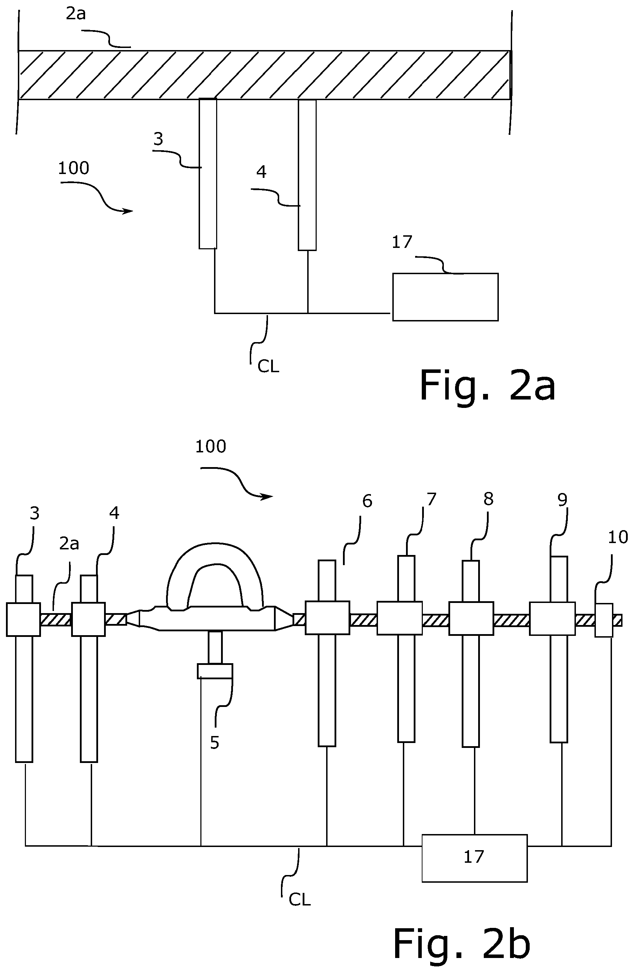

Fig 2a shows a schematic view of a first embodiment of the measuring system 100, comprising the UV-VIS spectrometer or spectrophotometer 3 and, again, a Raman spectrometer 4 for determining the total metal sulfate concentration c_MeSO4. The measuring system 100 is installed in a pipe measuring section of a pipe 2a of an industrial plant. The pipe 2a serves for guiding the solution 1, for example to a tank 2

The UV-VIS measuring spectrum as well as the Raman measuring spectrum are transmitted via a communication link CL to an evaluation and/or computation unit 17. The communication link CL is, for example, embodied as a wired communication connection, a wired automation technology fieldbus, e.g., Foundation Fieldbus, Profibus PA, Profibus DP, HART, Modbus RTU, CAN bus. However, it can also be a communication connection of a modern industrial communication network, e.g., an "Industrial Ethernet" fieldbus, in particular Profinet, Ethernet/IP, Modbus TCP, HART-IP, MQTT or OPC UA, especially applied according to their standard specification or a field bus version, in particular the IEEE 802.3cg-based standards such as Ethernet-APL or a communication network known from the communication sector, e.g., Ethernet according to the TCP/IP protocol. If the communication connection is wireless, it can be a Bluetooth, ZigBee, WLAN, GSM, LTE, UMTS communication network or a wireless version of a field bus, in particular 802.15.4-based standards such as WirelessHART.

By using the method according to the invention, the evaluation and/or computation unit 17 determines at least the nickel sulfate concentration c_NiSO4, the cobalt sulfate concentration c_CoSO4, and the manganese sulfate concentration c_MnSO4.

Fig. 2b shows a second embodiment of the measuring system 100, installed in-line in a measuring section of a pipe 2a. The measuring system 100 comprises various in-line measurement apparatuses 3, 4, 5, 6, 7, 8, 9, 10 which are in this embodiment combined in a compact manner. For example, the pipe measuring section in which the measuring system 100 is arranged has a length of less than 3 m in a longitudinal direction of the pipe.

The measuring system 100 of the embodiment in Fig. 2b comprises the following in-line measurement apparatuses 3, 4, 5, 6, 7, 8, 9, 10:
- the UV-VIS spectrometer or spectrophotometer 3,
- the Raman spectrometer 4,
- a mass density measuring apparatus 5,
- a refractometer 6,
- a conductivity sensor 7,
- an ORP sensor 8,
- a pH sensor 9, and
- a temperature sensor 10.

The mass density measuring apparatus 5 is here, advantageously of the Coriolis type. Preferably, it is embodied to measure the mass density, the mass flow and the viscosity, and e.g., the temperature.

Depending on the embodiment of the method, the above-mentioned in-line apparatuses may serve for determining the total metal sulfate concentration c_MeSO4, for example, the Raman spectrometer 4 and/or the mass density measuring apparatus 5, and/or, depending on the solution 1; 1a; 1b, the conductivity sensor 7.

Alternatively, the above-mentioned in-line apparatuses may serve for considering a cross-sensitivity on the determined metal sulfate concentrations of other measuring variables such as pH value and/or conductivity, by using for example the pH sensor 9 and the conductivity sensor 7, respectively.

Additionally, the above-mentioned in-line apparatuses may serve for first determining the presence of acid residuals such as H2O2 and H2SO4, and then considering the impact of these residuals on the determined metal sulfate concentrations. For example, the same UV-VIS spectrometer or spectrophotometer 3 may be also used to the H2O2 concentration, by analyzing the same UV-VIS measuring spectrum used for determining the cobalt sulfate concentration c_CoSO4 and nickel sulfate concentration c_NiSO4. The pH sensor 9 may be used to determine the concentration of H2SO4.

Additionally, the measuring system 100 may comprise an additional in-line adsorption spectrometer (not shown), for performing in-line Gamma-ray or X-ray absorption measurements, for determining the total fourth metal sulfate concentration c_MxSO4.

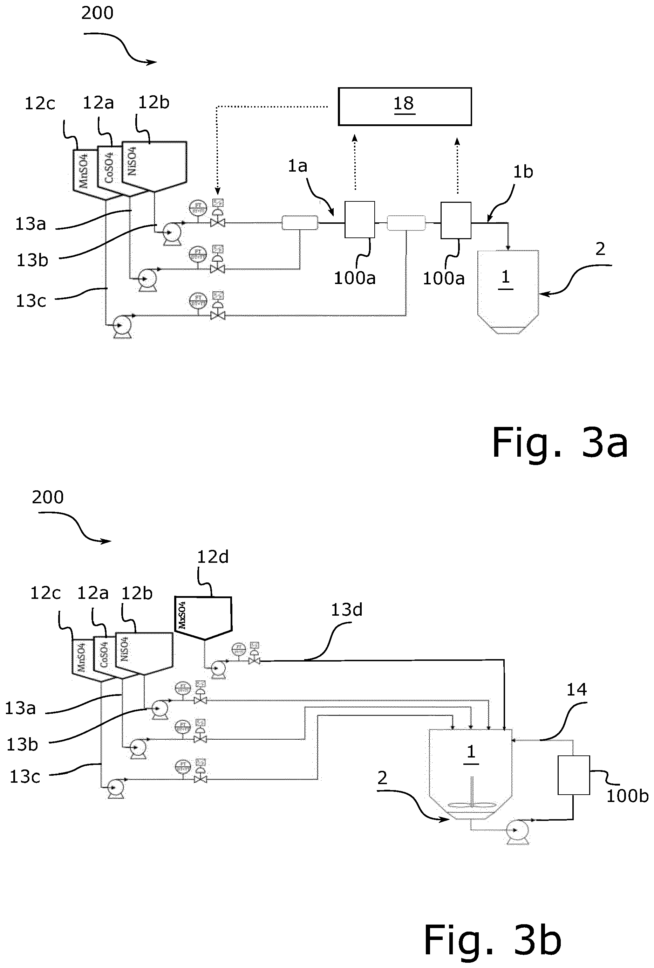

Finally, Fig. 3a-c show different embodiments of the installation assembly 200 comprising one or more of the measuring systems 100, 100a, 100b, 100c according to the invention. Of course, the embodiments shown in Fig.3a-c may be combined with one another. When comprising more than one measuring system 100, 100a, 100b, 100c, the installation assembly 200, may of course comprise different embodiments of the measuring system 100, 100a, 100b, 100c.

Starting with Fig. 3a, the installation assembly 200 comprises supply storages 12a, 12b, 12c for the first (CoSO4), the second (NiSO4), and the third metal sulfate (MnSO4), e.g., in form of a solution containing said metal sulfate. From each of these supply storages 12a,12b,12c, a respective supply pipe 13a,13b,13c is provided, for guiding the individual metal sulfate into a tank 2 containing the solution 1. The supply pipes 13a,13b,13c may each comprise a pump for conveying the metal sulfate, as well as a flowmeter and a valve unit, for controlling the amount of metal sulfate added to the solution 1; 1a; 1b on its way into the tank 2. Advantageously, as shown in Fig. 2a the supply pipes 13a, 13b, 13c are interconnected in such a way that the individual metal sulfates are mixed on their way from the supply storages 12a, 12b, 12c into the tank, by using static mixing.

A first measuring system 100 according to the invention is used before the third metal sulfate MnSO4 is added, and another first measuring system 100a is used after the admixing of the third metal sulfate MnSO4. In this way, the individual metal sulfate concentrations c_NiSO4, c_CoSO4, c_MnSO4 and the total metal sulfate concentration c_MeSO4 are determined before and after adding the third metal sulfate MnSO4.

Of course, alternatively, one single first measuring system 100a could be used to determine the individual metal sulfate concentrations c_NiSO4, c_CoSO4, c_MnSO4 and the total metal sulfate concentration c_MeSO4, both before and after admixing the third metal sulfate MnSO4, in case the installation assembly 200 comprises a topology of the pipe system suitable for this purpose, for example, by using one or more valve units.

In the embodiment show in Fig. 3a, the individual metal sulfates are in-line mixed with one another on their way to the tank 2. For example, static mixing may be used for the in-line blending. Thus, there is no need for an additional mixer in the tank 2. Preferably, the individual metal sulfate concentrations c_NiSO4, c_CoSO4, c_MnSO4 are determined at least between each in-line mixing step.

The determined concentrations c_NiSO4, c_CoSO4, c_MnSO4 may be transmitted to a control unit 18 of the installation assembly 200, which is for example associated with or embodied as a Control-programmed control unit (short: SPS) of the industrial plant, to control one or more of the valves associated with the supply pipes 13a,13b,13c. In such a way, one or more of the in-line determined concentrations c_NiSO4, c_CoSO4 and/or c_MnSO4 may be directly used as control parameters, for reaching required, preset values for the individual metal sulfate concentrations.

The second embodiment shown in Fig. 3b differs from the one of Fig. 3a only slightly. A fourth metal sulfate MxSO4 contained in a fourth supply storage 12d, is added to the solution 1, wherein the atomic number of the metal Mx differs by at least 5, especially at least by 10 from the atomic numbers of the metals of the first, second and third metal sulfates, namely Co, Ni and Mn. Additionally, in this embodiment, the tank 2 comprising the solution 1 contains a mixer. Of course, the in-line mixing shown in Fig. 3a could also be used for this embodiment, instead of or in combination with the mixer arranged in the tank 2. Similarly, the fourth metal sulfate MxSO4 and its fourth supply storage 12d, may also be combined with the embodiment shown in Fig. 3a.

In this case, a second measuring system 100b is used, which is arranged in a circuit pipe 14 leading into and out of the tank 2. The circuit pipe 14 comprises also a pump for conveying the solution 1 in the circuit pipe. The second measuring system 100b now comprises a suitable in-line adsorption spectrometer, which is embodied to perform in-line Gamma-ray or X-ray absorption measurements, especially using a low energy gamma or X-ray source, having energies below 350 keV, especially below 200 keV, and obtaining a Gamma-ray or X-ray measuring spectrum. By analyzing the Gamma-ray or X-ray measuring spectrum, the concentration of the fourth metal sulfate c_MxSO4 is determined. The manganese sulfate concentration c_MnSO4 contained in the solution 1 is, again, indirectly determined, based on the nickel sulfate concentration c_NiSO4, the cobalt sulfate concentration c_CoSO4 and the total metal sulfate concentration c_MeSO4. Also, even if not explicitly shown in Fig. 3b, the determined metal sulfate concentrations c_NiSO4, c_CoSO4, c_MnSO4 and/or MxSO4, may be used, similarly to the embodiment shown in Fig. 3a, as control parameters for the installation assembly 200 of the industrial plant.

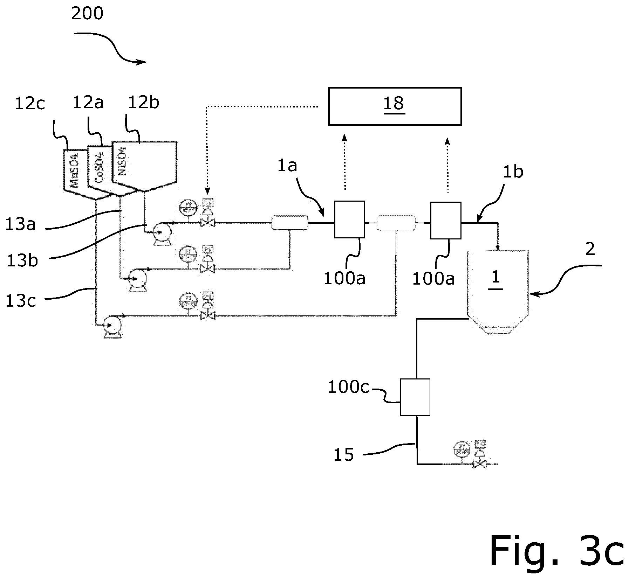

Finally, in Fig. 3c, a third measuring system 100c is installed in a feeding pipe 15, which leads to a crystallizer for crystallizing NMC-based cathode materials. Also, the feeding pipe 15 may comprise a valve unit, whose setting depends on the metal sulfate concentrations c_NiSO4, c_CoSO4, c_MnSO4 in-line determined by the third measuring system 100c. Thus, only when the determined metal sulfate concentrations c_NiSO4, c_CoSO4, c_MnSO4 are within a specified, acceptable range, the solution 1 is in fact provided to the crystallizer, and will be otherwise discarded or, for example, returned into the tank 2.

### Reference Signs and Symbols

- 1, 1a, 1b: Solutions
- 2a,2b,2c,...: Pipes
- 2: Tank
- 3: UV-VIS spectrometer or spectrophotometer
- 4: Raman spectrometer
- 5: mass density measuring apparatus
- 6: refractometer
- 7: conductivity sensor
- 8: ORP sensor
- 9: pH sensor
- 10: temperature sensor
- 11: laboratory spectrometer
- 100, 100a, 100b, 100c: Measuring system
- 200: Installation Assembly
- 12a,12b,12c,12d: Supply storages
- 13a, 13b, 13c, 13c: Supply pipes
- 14: Circuit pipe
- 15: Feeding pipe
- 16: Model
- 17: evaluation and/or computation unit
- 18: control unit

- CL: communication link

- c_CoSO4: Cobalt Sulfate concentration
- c_NiSO4: Nickel Sulfate concentration
- c_MnSO4: Manganese Sulfate concentration
- c_MeSO4: Total Metal Sulfate concentration
- c_MxSO4: Fourth Metal Sulfate concentration

## Claims

1. Method for in-line monitoring the concentration of at least NiSO4, CoSO4 and MnSO4 in a solution (1;1a,1b;...) comprising at least one metal sulfate, wherein the solution (1;1a,1b;...) is contained in a pipe (2a;2b;2c,...) or tank (2) of an industrial plant, the method comprising:
- In-line obtaining a UV-VIS measuring absorption and/or reflectance spectrum of the solution (1;1a,1b;...), especially by using ultraviolet or ultraviolet-visible or near infrared electromagnetic waves having a wavelength within a range from 200 nm to 1000 nm, preferably 300 nm to 900 nm,
- In-line determining the concentration of CoSO4 (c_CoSO4) as a first metal sulfate contained in the solution (1;1a,1b;...) and the concentration of NiSO4 (c_NiSO4) as a second metal sulfate contained in the solution (1;1a,1b;...), based at least on an in-line analysis of the UV-VIS measuring spectrum,
- In-line determining the total metal sulfate concentration (c_MeSO4) contained in the solution (1;1a,1b;...), by using at least one in-line measurement which is sensitive to the total concentration (c_MeSO4) of the metal sulfates,
wherein the in-line determination of the total metal sulfate concentration (c_MeSO4) is performed in addition to the in-line obtaining of the UV-VIS measuring spectrum, by using a method different from UV-VIS absorption and/or reflectance;
- In-line determining the concentration of MnSO4 (c_MnSO4) as a third metal sulfate contained in the solution (1;1a,1b;...), based at least on the determined concentration of NiSO4 (c_NiSO4), the determined concentration of CoSO4 (c_CoSO4), and the determined total metal sulfate concentration (c_MeSO4).

2. Method according to Claim 1,
wherein in the industrial plant, MnSO4 is admixed to a first solution (1a) comprising NiSO4 and CoSO4 as metal sulfates, yielding a second (1b) solution,
wherein the determining of the total metal sulfate concentration (c_MeSO4) is carried out both with the first solution (1a) and with the second solution (1b),
and wherein the determining of the concentration of MnSO4 (c_MnSO4) in the second solution (1b) is based at least on:
- the determined concentration of NiSO4 (c_NiSO4) of the first solution (1a) and/or the determined concentration of NiSO4 (c_NiSO4) of the second solution (1b),
- the determined concentration of CoSO4 (c_CoSO4) of the first solution (1a) and/or the determined concentration of CoSO4 (c_CoSO4) of the second solution (1b), and
- the determined total metal sulfate concentration (c_MeSO4) of the first solution (1a) and the determined total metal sulfate concentration (c_MeSO4) of the second solution (1b).

3. Method according to at least one of the previous claims,
wherein the at least one in-line measurement for determining the total metal sulfate concentration (c_MeSO4) comprises at least one of the following:
- Raman spectroscopy carried out with an in-line Raman spectrometer (4), wherein a Raman measuring spectrum is in-line analyzed to determine the total concentration of all metal sulfates in the solution (1;1a,1b;...),
- A total mass density measurement carried out with an in-line mass density measuring apparatus (5),
- A Refractive index measurement carried out with an in-line refractometer (6).

4. Method according to at least one of the previous claims,
wherein the solution (1;1a,1b;...) contains a fourth metal sulfate (MxSO4) and the atomic number of the metal (Mx) of the fourth metal sulfate (MxSO4) differs by at least 5, especially at least by 10 from the atomic numbers of the metals of the first, second and third metal sulfates, namely Co, Ni and Mn, the method comprising:
- Performing in-line Gamma-ray or X-ray absorption measurements, especially using a low energy gamma or X-ray source, having energies below 350 keV, especially below 200 keV, and obtaining a Gamma-ray or X-ray measuring spectrum,
- In-line determining the concentration of the fourth metal sulfate (c_MxSO4), by analyzing the Gamma-ray or X-ray measuring spectrum, and
- In-line determining the concentration of MnSO4 as a third metal sulfate contained in the solution (1;1a,1b;...),
based at least on the determined concentration of NiSO4 (c_NiSO4), the determined concentration of CoSO4 (c_CoSO4), the determined concentration of the fourth metal sulfate (c_MxSO4), and the determined total metal sulfate concentration (c_MeSO4).

5. Method according to at least one of the previous claims,
wherein in case the solution (1;1a,1b;...) contains less than 500ppm, especially less than 100 ppm, of other ions which are no metal sulfates, in relation to the total ion concentration, the at least one measurement method for determining the total metal sulfate concentration (c_MeSO4) comprises an in-line conductivity measurement, carried out with an in-line conductivity sensor (7).

6. Method according to at least one of the previous claims, comprising
- In-line determining the concentration of H2O2, by in-line analyzing the UV-VIS measuring spectrum and/or by in-line analyzing the oxidation reduction potential measured in-line with an in-line oxidation reduction potential sensor (8), and/or
- In-line determining the concentration of H2SO4, by in-line measuring the pH- value (9), and
- Considering the determined H2O2 concentration and/or the determined H2O4 concentration, in the determining of the total (c_MeSO4) and/or individual metal sulfate concentration (c_CoSO4;c_NiSO4; c_MnSO4; c_MxSO4) of the first, second, third, and, if applicable, fourth metal sulfate.

7. Method according to at least one of the previous claims, comprising:
- In-line determining the temperature of the solution (1;1a,1b;...), and
- Considering the determined temperature, in the determining of the total (c_MeSO4) and/or individual metal sulfate concentration (c_CoSO4; c_NiSO4; c_MnSO4; c_MxSO4) of the first, second, third, and, if applicable, fourth metal sulfate.

8. Method according to at least one of the previous claims,
wherein the in-line determination of the concentration of the first, the second, the third, and, if applicable, the fourth metal sulfate concentration (c_CoSO4;c_NiSO4; c_MnSO4; c_MxSO4) comprises:
In-line performing a, especially multivariate, analysis, wherein the analysis includes the processing of at least two of the following:
- the UV-VIS measuring spectrum, the Raman measuring spectrum, the total mass density, the Gamma-ray or X-ray measuring spectrum, the pH-value, the conductivity, the oxidation reduction potential, the determined concentration of H2SO4, the determined concentration of H2O2, the determined temperature.

9. Method according to Claim 8,
wherein a model (16) used in the, especially multivariate, analysis is calibrated, verified, adjusted and/or trained by using a laboratory spectrometer (31), especially an inductively coupled plasma atomic emission spectroscopy spectrometer in a laboratory located in the industrial plant or remote from the industrial plant.

10. In-line Measuring system (100), wherein the in-line measuring system (100) is embodied to carry out the method according to at least one of the previous claims,
wherein the measuring systems (100) comprises:
- an in-line ultraviolet or ultraviolet-visible absorption and/or reflectance spectrometer or spectrophotometer (3), for in-line determining an ultraviolet-visible or ultraviolet absorption and/or reflectance spectrum,
- at least an in-line measuring apparatus (4; 5; 6; 7) different from the ultraviolet or ultraviolet-visible absorption and/or reflectance spectrometer or spectrophotometer, wherein the in-line measuring apparatus is embodied to in-line determine the total metal sulfate concentration (c_MeSO4) contained in the solution (1; 1a, 1b;...), by using at least one measurement method which is sensitive to the total concentration of the metal sulfates, and
- an evaluation and/or computation unit, embodied to determine the concentration of at least NiSO4, CoSO4 and MnSO4 (c_CoSO4, c_NiSO4, c_MnSO4) in the solution (1; 1a, 1b;...).

11. Installation assembly (200) for producing a solution (1;1a,1b;...) containing metal sulfates in an industrial plant, comprising
- a tank (2) for containing the solution (1)
- a pipe system comprising pipes (2a;2b;2c;...) for guiding the solution (1;1a,1b;...), wherein the pipes (2a;2b;2c;...) lead into or out of the tank (2), and
- at least a first in-line measuring (100) system as claimed in Claim 10, wherein the first in-line measuring (100) system is installed in the pipe system.

12. Installation assembly (200) according to Claim 11, comprising:
- a first, second, third, and, if applicable, fourth supply storage (12a,12b,12c;12d) for the first, second, third, and, if applicable, fourth metal sulfate, respectively,
- supply pipes (13a,13b,13c;13d) leading from the first, second, third, and, if applicable, fourth supply storage to the tank (2), for producing the solution (1;1a,1b;...),
wherein the Installation assembly (200) is designed in such a way that the concentration of the first, second, third, and if applicable, fourth metal sulfate (c_CoSO4,c_NiSO4, c_MnSO4; c_MxSO4) in the solution (1;1a,1b;...) is determined with at least the first In-line Measuring system (100), on its way from its respective supply storage (12a;12b;12c;12d) into the tank (2) during the admixing of the respective metal sulfate to the solution (1;1a,1b;...),
and wherein especially the installation assembly (200) is embodied to in-line blend the first, second, third, and, if applicable, fourth metal sulfate with one another, on their way from their respective supply storage (12a;12b;12c;12d) into the tank (2), preferably using static mixing.

13. Installation Assembly (200) according to at least one of the Claims 11 or 12, comprising a second in-line measuring system (100b) as claimed in Claim 10, wherein the second in-line measuring (100b) system is arranged in a circuit pipe (14), wherein the circuit pipe (14) is leading from a first outlet leading out of the tank (2) to an inlet leading into of the tank (2) and/or
comprising a third in-line measuring system (100c) as claimed in Claim 10, wherein the third in-line measuring system (100c) is arranged with respect to a feeding pipe (15), which feeding pipe (15) leads from a second outlet of the tank to a crystallizer in such a way that the concentration of the first, second, third, and if applicable, fourth metal sulfate (c_CoSO4,c_NiSO4, c_MnSO4; c_MxSO4) in the solution (1;1a,1b;...) is determined on its way from the tank (2) to the crystallizer.

14. Installation Assembly (200) according to at least one of the Claims 11 to 13,
wherein the installation assembly (200) is embodied to control the inflow from the supply pipes (13a,13b,13c;13d) into the tank (2), based on the concentration of the first metal sulfate, the second metal sulfate, the third metal sulfate, and/or, if applicable, the fourth metal sulfate (c_CoSO4,c_NiSO4, c_MnSO4; c_MxSO4), determined in-line with the first in-line measuring system (100), the second in-line measuring system (100b) and/or the third in-line measuring system (100c).

15. Use of the installation assembly (200) according to any of the Claims 11 to 14, in a feeding pipe (15) for a crystallizer for crystallization of cathode materials for lithium-ion batteries.

## Patentansprüche

1. Methode zur Inline-Überwachung der Konzentration von mindestens NiSO4, CoSO4 und MnSO4 in einer Lösung (1, 1a, 1b ...), die mindestens ein Metallsulfat enthält, wobei sich die Lösung (1, 1a, 1b ...) in einem Rohr (2a, 2b, 2c ...) oder Tank (2) einer Industrieanlage befindet und die Methode Folgendes umfasst:
- Inline-Ermittlung eines UV-VIS-Absorptions- und/oder Reflexionsmessspektrums der Lösung (1, 1a, 1b ...), insbesondere durch Nutzung von elektromagnetischen Wellen im ultravioletten oder ultraviolett-sichtbaren oder Nahinfrarotbereich, die eine Wellenlänge im Bereich von 200 nm bis 1000 nm, und vorzugsweise zwischen 300 nm und 900 nm, aufweisen.
- Inline-Bestimmung der Konzentration von CoSO4 (c_CoSO4) als erstes in der Lösung (1, 1a, 1b ...) enthaltenes Metallsulfat und der Konzentration von NiSO4 (c_NiSO4) als zweites in der Lösung (1, 1a, 1b ...) enthaltenes Metallsulfat, basierend auf mindestens einer Inline-Analyse des UV-VIS-Messspektrums.
- Inline-Bestimmung der in der Lösung (1, 1a, 1b ...) enthaltenen Gesamtkonzentration an Metallsulfaten (c_MeSO4) durch Nutzung von mindestens einer Inline-Messung, die empfindlich auf die Gesamtkonzentration (c_MeSO4) an Metallsulfaten reagiert, wobei die Inline-Bestimmung der Gesamtkonzentration an Metallsulfaten (c_MeSO4) zusätzlich zur Inline-Ermittlung des UV-VIS-Spektrums durchgeführt wird, und zwar mithilfe einer anderen Methode als der UV-VIS-Absorption und/oder -Reflexion.
- Inline-Bestimmung der Konzentration von MnSO4 (c_MnSO4) als drittes in der Lösung (1, 1a, 1b ...) enthaltenes Metallsulfat, basierend auf mindestens der ermittelten Konzentration von NiSO4 (c_NiSO4), der ermittelten Konzentration von CoSO4 (C_CoSO4) und der ermittelten Gesamtkonzentration an Metallsulfaten (c_MeSO4).

2. Methode gemäß Anspruch 1, wobei in der Industrieanlage MnSo4 einer ersten Lösung (1a), die NiSO4 und CoSO4 als Metallsulfate enthält, beigemischt wird, sodass sich eine zweite (1b) Lösung ergibt, wobei die Bestimmung der Gesamtkonzentration an Metallsulfaten (c_MeSO4) sowohl mit der ersten Lösung (1a) als auch mit der zweiten Lösung (1b) durchgeführt wird und wobei die Bestimmung der Konzentration von MnSO4 (c_MnSO4) in der zweiten Lösung (1b) mindestens auf Folgendem basiert:
- Der ermittelten Konzentration von NiSO4 (c_NisO4) in der ersten Lösung (1a) und/oder der ermittelten Konzentration von NiSO4 c_NiSO4) in der zweiten Lösung (1b)
- Der ermittelten Konzentration von CoSO4 (c_CoSO4) in der ersten Lösung (1a) und/oder der ermittelten Konzentration von CoSO4 (c_CoSO4) in der zweiten Lösung (1b) und
- Der ermittelten Gesamtkonzentration an Metallsulfaten (c_MeSO4) in der ersten Lösung (1a) und der ermittelten Gesamtkonzentration an Metallsulfaten (c_MeSO4) in der zweiten Lösung (1b)

3. Methode gemäß mindestens einem der vorangehenden Ansprüche, wobei die mindestens eine Inline-Messung zur Bestimmung der Gesamtkonzentration an Metallsulfaten (c_MeSO4) mindestens eins der folgenden Elemente umfasst:
- Raman-Spektroskopie, die mit einem Inline-Raman-Spektrometer (4) durchgeführt wird, wobei ein Raman-Messspektrum inline analysiert wird, um die Gesamtkonzentration aller Metallsulfate in der Lösung (1, 1a, 1b ...) zu bestimmen
- Eine Messung zur Ermittlung der Gesamtmassendichte, die mit einem Inline-Massendichte-Messgerät (5) durchgeführt wird
- Eine Messung des Brechungsindexes, die mit einem Inline-Refraktometer (6) durchgeführt wird

4. Methode gemäß mindestens einem der vorangehenden Ansprüche, wobei die Lösung (1, 1a, 1b ...) ein viertes Metallsulfat (MxSO4) enthält und die Ordnungszahl des Metalls (Mx) des vierten Metallsulfats (MxSO4) um mindestens 5, insbesondere um mindestens 10 von den Ordnungszahlen der Metalle des ersten, zweiten und dritten Metallsulfats, d. h. Co, Ni und Mn, abweicht, wobei die Methode Folgendes umfasst:
- Durchführung von Inline-Gammastrahlen- oder Röntgenstrahlen-Absorptionsmessungen, insbesondere unter Verwendung einer niedrigenergetischen Gamma- oder Röntgenstrahlungsquelle, deren Energie unter 350 keV liegt, insbesondere unter 200 keV, und Erstellung eines Gammastrahlen- oder Röntgenstrahlen-Messspektrums.
- Inline-Bestimmung der Konzentration des vierten Metallsulfats (c_MxSO4) durch Analyse des Gammastrahlen- oder Röntgenstrahlen-Messspektrums und
- Inline-Bestimmung der Konzentration von MnSO4 als drittes in der Lösung (1, 1a, 1b ...) enthaltenes Metallsulfat, und zwar auf der Basis der ermittelten Konzentration von NiSO4 (c_NiSO4), der ermittelten Konzentration von CoSO4 (c_CoSO4), der ermittelten Konzentration des vierten Metallsulfats (c_MxSO4) und der ermittelten Gesamtkonzentration an Metallsulfaten (c_MeSO4).

5. Methode gemäß mindestens einem der vorangehenden Ansprüche, wobei in dem Fall, dass die Lösung (1, 1a, 1b ...) im Verhältnis zur Gesamtionenkonzentration weniger als 500 ppm, und insbesondere weniger als 100 ppm, der anderen Ionen enthält, bei denen es sich nicht um Metallsulfate handelt, die mindestens eine Messmethode zur Bestimmung der Gesamtkonzentration an Metallsulfaten (c_MeSo4) eine Inline-Leitfähigkeitsmessung umfasst, die mit einem Inline-Leitfähigkeitssensor (7) ausgeführt wird.

6. Methode gemäß mindestens einem der vorangehenden Ansprüche, die Folgendes umfasst:
- Inline-Bestimmung der Konzentration von H2O2 durch Inline-Analyse des UV-VIS-Messspektrums und/oder durch Inline-Analyse des Redoxpotentials, das inline mit einem Inline-Sensor für das Redoxpotential (8) gemessen wurde und/oder
- Inline-Bestimmung der Konzentration von H2SO4 durch Inline-Messung des pH-Werts (9) und
- Berücksichtigung der ermittelten H2O2-Konzentration und/oder der ermittelten H2O4-Konzentration bei der Bestimmung der Gesamt- (c_MeSO4) und/oder der einzelnen Metallsulfatkonzentration (c_CoSO4, c_NiSO4, c_MnSO4, c_MxSO4) des ersten, zweiten, dritten und, gegebenenfalls, vierten Metallsulfats.

7. Methode gemäß mindestens einem der vorangehenden Ansprüche, die Folgendes umfasst:
- Inline-Bestimmung der Temperatur der Lösung (1, 1a, 1b ...) und
- Berücksichtigung der ermittelten Temperatur bei der Bestimmung der Gesamt- (c_MeSO4) und/oder individuellen Metallsulfatkonzentration (c_CoSO4, c_NiSO4, c_MnSO4, c_MxSO4) des ersten, zweiten, dritten und, gegebenenfalls, vierten Metallsulfats.

8. Methode gemäß mindestens einem der vorangehenden Ansprüche, wobei die Inline-Bestimmung der Konzentration der ersten, zweiten, dritten und, gegebenenfalls, vierten Metallsulfatkonzentration (c_CoSO4, c_NiSO4, c_MnSO4, c_MxSO4) Folgendes umfasst:
Inline-Durchführung einer, insbesondere multivariaten, Analyse, wobei die Analyse die Verarbeitung von mindestens zwei der folgenden Werte umfasst:
- Das UV-VIS-Messspektrum, das Raman-Messspektrum, die gesamte Massendichte, das Gammastrahlen- oder Röntgenstrahlen-Messspektrum, der pH-Wert, die Leitfähigkeit, das Redox-Potential, die ermittelte Konzentration von H2SO4, die ermittelte Konzentration von H2O2, die ermittelte Temperatur.

9. Methode gemäß Anspruch 8, wobei ein bei der, insbesondere multivariaten, Analyse verwendetes Modell (16) kalibriert, verifiziert, angepasst und/oder eingelernt wird und zwar mithilfe eines Laborspektrometers (31), insbesondere eines Spektrometers für die induktiv gekoppelte Plasma-Atomemissionsspektroskopie in einem Labor, das sich in der industriellen Anlage oder entfernt von der industriellen Anlage befindet.

10. Inline-Messsystem (100), wobei das Inline-Messsystem (100) dafür ausgelegt ist, die Methode gemäß mindestens einem der vorangehenden Ansprüche durchzuführen, wobei das Messsystem (100) Folgendes umfasst:
- Ein Inline-Absorptions- und/oder Reflexionsspektrometer oder Spektrophotometer (3) für den ultravioletten oder ultraviolett-sichtbaren Bereich, das zur Inline-Ermittlung des ultraviolett-sichtbaren oder ultravioletten Absorptions- und/oder Reflexionsspektrums dient
- Und mindestens ein Inline-Messgerät (4, 5, 6, 7), das sich vom Absorptions- und/oder Reflexionsspektrometer oder Spektrophotometer für den ultravioletten oder ultraviolett-sichtbaren Bereich unterscheidet, wobei das Inline-Messgerät dafür ausgelegt ist, inline die in der Lösung (1, 1a, 1b ...) enthaltene Gesamtkonzentration an Metallsulfaten (c_MeSO4) zu ermitteln, indem es mindestens eine Messmethode nutzt, die empfindlich auf die Gesamtkonzentration der Metallsulfate reagiert, sowie eine Auswerte- und/oder Recheneinheit, die darauf ausgelegt ist, die Konzentration von mindestens NiSO4, CoSO4 und MnSO4 (c_CoSO4, c_NiSO4, c_MnSO4) in der Lösung (1, 1a, 1b ...) zu bestimmen.

11. Montagebaugruppe (200) zur Erzeugung einer Lösung (1, 1a, 1b ...), die Metallsulfate in einer Industrieanlage enthält und Folgendes umfasst:
- Einen Tank (2) zur Aufnahme der Lösung (1)
- Ein Rohrleitungssystem bestehend aus Rohren (2a, 2b, 2c ...) zur Führung der Lösung (1, 1a, 1b ...), wobei die Rohre (2a, 2b, 2c ...) in den oder aus dem Tank (2) führen und
- Mindestens ein erstes Inline-Messsystem (100) gemäß Anspruch 10, wobei das erste Inline-Messsystem (100) im Rohrleitungssystem installiert ist.

12. Montagebaugruppe (200) gemäß Anspruch 11, die Folgendes umfasst:
- Einen ersten, zweiten, dritten und, gegebenenfalls, vierten Vorratsbehälter (12a, 12b, 12c, 12d) für das erste bzw. das zweite, dritte und, gegebenenfalls, vierte Metallsulfat
- Versorgungsrohrleitungen (13a, 13b, 13c, 13d), die zur Herstellung der Lösung (1, 1a, 1b ...) vom ersten, zweiten, dritten und, gegebenenfalls, vierten Versorgungsbehälter zum Tank (2) führen,
wobei die Montagebaugruppe (200) so ausgelegt ist, dass die Konzentration des ersten, zweiten, dritten und, gegebenenfalls, vierten Metallsulfats (c_CoSO4, c_NiSO4, c_MnSO4, c_MxSO4) in der Lösung (1, 1a, 1b ...) auf dem Weg von dem jeweiligen Vorratsbehälter (12a, 12b, 12c, 12d) in den Tank (2) während der Beimischung des jeweiligen Metallsulfats zur Lösung (1, 1a, 1b ...) mit mindestens dem ersten Inline-Messsystem (100) ermittelt wird und wobei insbesondere die Montagebaugruppe (200) dafür ausgelegt ist, das erste, zweite, dritte und, gegebenenfalls, vierte Metallsulfat inline auf dem Weg von seinem jeweiligen Vorratsbehälter (12a, 12b, 12c, 12d) in den Tank (2) mit den jeweils anderen zu mischen und zwar vorzugsweise mittels statischer Mischung.

13. Montagebaugruppe (200) gemäß mindestens einem der Ansprüche 11 oder 12, die ein zweites, wie in Anspruch 10 aufgeführtes Inline-Messsystem (100b) umfasst, wobei das zweite Inline-Messsystem (100b) in einem Rohrkreislauf (14) angeordnet ist, wobei der Rohrkreislauf (14) von einem ersten Auslass, der aus dem Tank (2) führt, zu einem Einlass, der in den Tank (2) führt, verläuft
und/oder
ein drittes Inline-Messsystem (100c) gemäß Anspruch 10 umfasst, wobei das dritte Inline-Messsystem (100c) in Bezug auf ein Zuleitungsrohr (15) angeordnet ist, wobei das Zuleitungsrohr (15) von einem zweiten Auslass des Tanks zu einem Kristallisator führt und zwar so, dass die Konzentration des ersten, zweiten, dritten und, gegebenenfalls, vierten Metallsulfats (c_CoSO4, c_NiSO4, c_MnSO4, c_MxSO4) in der Lösung (1, 1a, 1b ...) auf dem Weg vom Tank (2) zum Kristallisator ermittelt wird.

14. Montagebaugruppe (200) gemäß mindestens einem der Ansprüche 11 bis 13, wobei die Montagebaugruppe (200) dafür ausgelegt ist, den Zufluss von den Versorgungsleitungen (13a, 13b, 13c, 13d) in den Tank (2) zu steuern, und zwar basierend auf der Konzentration des ersten Metallsulfats, des zweiten Metallsulfats, des dritten Metallsulfats und/oder, gegebenenfalls, des vierten Metallsulfats (c_CoSO4, c_NiSO4, c_MnSO4, c_MxSO4), das inline mit dem ersten Inline-Messsystem (100), dem zweiten Inline-Messsystem (100b) und/oder dem dritten Inline-Messsystem (100c) ermittelt wurde.

15. Verwendung der Montagebaugruppe (200) gemäß einem der Ansprüche 11 bis 14 in einem Zuleitungsrohr (15) für einen Kristallisator zur Kristallisation der Kathodenmaterialien für Lithium-Ionen-Batterien.

## Revendications

1. Procédé destiné à la surveillance en ligne de la concentration au minimum en NiSO4, CoSO4 et MnSO4 dans une solution (1, 1a, 1b, ...) comprenant au moins un sulfate métallique, la solution (1, 1a, 1b, ...) étant contenue dans une conduite (2a, 2b, 2c, ...) ou dans une cuve (2) d'une installation industrielle, le procédé comprenant les étapes suivantes :
- Acquisition en ligne d'un spectre de mesure de l'absorbance et/ou de la réflectance UV-VIS de la solution (1, 1a, 1b, ...), notamment à l'aide d'ondes électromagnétiques ultraviolettes, ultraviolettes-visibles ou du proche infrarouge, lesquelles ondes présentent une longueur d'onde comprise dans une gamme allant de 200 nm à 1 000 nm, de préférence de 300 nm à 900 nm,
- Détermination en ligne de la concentration en CoSO4 (c_CoSO4) en tant que premier sulfate métallique contenu dans la solution (1, 1a, 1b, ...) et de la concentration en NiSO4 (c_NiSO4) en tant que deuxième sulfate métallique contenu dans la solution (1, 1a, 1b, ...), sur la base d'au moins une analyse en ligne du spectre de mesure UV-VIS,
- Détermination en ligne de la concentration totale en sulfates métalliques (c_MeSO4) contenue dans la solution (1, 1a, 1b, ...) à l'aide d'au moins une mesure en ligne sensible à la concentration totale (c_MeSO4) en sulfates métalliques, la détermination en ligne de la concentration totale en sulfates métalliques (c_MeSO4) étant effectuée en plus de l'acquisition en ligne du spectre de mesure UV-VIS, à l'aide d'une méthode différente de l'absorbance et/ou de la réflectance UV-VIS ;
- Détermination en ligne de la concentration en MnSO4 (c_MnSO4) en tant que troisième sulfate métallique contenu dans la solution (1, 1a, 1b, ...), sur la base, d'au moins la concentration déterminée en NiSO4 (c_NiSO4), de la concentration déterminée en CoSO4 (c_CoSO4) et de la concentration totale déterminée en sulfates métalliques (c_MeSO4).

2. Procédé selon la revendication 1,
pour lequel, dans l'installation industrielle, du MnSO4 est mélangé à une première solution (1a) comprenant du NiSO4 et du CoSO4 en tant que sulfates métalliques, obtenant ainsi une deuxième solution (1b),
la détermination de la concentration totale en sulfates métalliques (c_MeSO4) étant effectuée à la fois sur la première solution (1a) et sur la deuxième solution (1b),
et la détermination de la concentration en MnSO4 (c_MnSO4) dans la deuxième solution (1b) reposant au moins sur :
- la concentration déterminée en NiSO4 (c_NiSO4) de la première solution (1a) et/ou la concentration déterminée en NiSO4 (c_NiSO4) de la deuxième solution (1b),
- la concentration déterminée en CoSO4 (c_CoSO4) de la première solution (1a) et/ou la concentration déterminée en CoSO4 (c_CoSO4) de la deuxième solution (1b), et
- la concentration totale déterminée en sulfates métalliques (c_MeSO4) de la première solution (1a) et la concentration totale déterminée en sulfates métalliques (c_MeSO4) de la deuxième solution (1b).

3. Procédé selon au moins l'une des revendications précédentes,
pour lequel l'au moins une mesure en ligne destinée à déterminer la concentration totale en sulfates métalliques (c_MeSO4) comprend au moins l'une des étapes suivantes :
- Spectroscopie Raman réalisée à l'aide d'un spectromètre Raman en ligne (4), étape dans laquelle un spectre de mesure Raman est analysé en ligne afin de déterminer la concentration totale de tous les sulfates métalliques présents dans la solution (1 ; 1a, 1b, ...),
- Mesure de la densité massique totale réalisée à l'aide d'un appareil de mesure de densité massique en ligne (5),
- Mesure de l'indice de réfraction réalisée à l'aide d'un réfractomètre en ligne (6).

4. Procédé selon au moins l'une des revendications précédentes,
pour lequel la solution (1, 1a, 1b, ...) contient un quatrième sulfate métallique (MxSO4) et le numéro atomique du métal (Mx) du quatrième sulfate métallique (MxSO4) diffère d'au moins 5, notamment d'au moins 10, des numéros atomiques des métaux du premier, du deuxième et du troisième sulfate métallique, à savoir Co, Ni et Mn, le procédé comprenant les étapes suivantes :
- Réalisation de mesures en ligne de l'absorbance de rayons gamma ou de rayons X, notamment à l'aide d'une source de rayons gamma ou de rayons X de faible énergie, source pour laquelle les énergies sont inférieures à 350 keV, notamment inférieures à 200 keV, et l'obtention d'un spectre de mesure de rayons gamma ou de rayons X,
- Détermination en ligne de la concentration en sulfate du quatrième métal (c_MxSO4), par analyse du spectre de mesure des rayons gamma ou des rayons X, et
- Détermination en ligne de la concentration en MnSO4 en tant que sulfate d'un troisième métal contenu dans la solution (1, 1a, 1b, ...),
sur la base au moins de la concentration déterminée en NiSO4 (c_NiSO4), la concentration déterminée en CoSO4 (c_CoSO4), la concentration déterminée en sulfate du quatrième métal (c_MxSO4), et la concentration totale déterminée en sulfates métalliques (c_MeSO4).

5. Procédé selon au moins l'une des revendications précédentes,
pour lequel, dans le cas où la solution (1, 1a, 1b, ...) contient moins de 500 ppm, notamment moins de 100 ppm, d'autres ions qui ne sont pas des sulfates métalliques, par rapport à la concentration ionique totale, l'au moins un procédé de mesure permettant de déterminer la concentration totale en sulfates métalliques (c_MeSO4) comprend une mesure de conductivité en ligne, réalisée à l'aide d'un capteur de conductivité en ligne (7).

6. Procédé selon au moins l'une des revendications précédentes, comprenant les étapes suivantes :
- Détermination en ligne de la concentration en H2O2, par analyse en ligne du spectre de mesure UV-VIS et/ou par analyse en ligne du potentiel redox mesuré en ligne à l'aide d'un capteur de redox en ligne (8), et/ou
- Détermination en ligne de la concentration en H2SO4, par la mesure en ligne de la valeur du pH (9), et
- Prise en compte de la concentration déterminée en H2O2 et/ou de la concentration déterminée en H2O4, lors de la détermination de la concentration totale (c_MeSO4) et/ou des concentrations individuelles en sulfates métalliques (c_CoSO4, c_NiSO4, c_MnSO4, c_MxSO4) du premier, du deuxième, du troisième et, le cas échéant, du quatrième sulfate métallique.

7. Procédé selon au moins l'une des revendications précédentes, lequel procédé comprend les étapes suivantes :
- Détermination en ligne de la température de la solution (1, 1a, 1 b, ...), et
- Prise en compte de la température déterminée pour la détermination de la concentration totale (c_MeSO4) et/ou des concentrations individuelles (c_CoSO4, c_NiSO4, c_MnSO4, c_MxSO4) du premier, du deuxième, du troisième et, le cas échéant, du quatrième sulfate métallique.

8. Procédé selon au moins l'une des revendications précédentes,
pour lequel la détermination en ligne de la concentration du premier, du deuxième, du troisième et, le cas échéant, du quatrième sulfate métallique (c_CoSO4, c_NiSO4, c_MnSO4, c_MxSO4) comprend l'étape suivante :
Réalisation en ligne d'une analyse, notamment multivariée, l'analyse comprenant le traitement d'au moins deux des éléments suivants :
- le spectre de mesure UV-VIS, le spectre de mesure Raman, la densité massique totale, le spectre de mesure des rayons gamma ou des rayons X, le pH, la conductivité, le potentiel redox, la concentration déterminée en H2SO4, la concentration déterminée en H2O2, la température déterminée.

9. Procédé selon la revendication 8,
pour lequel un modèle (16) utilisé dans l'analyse, notamment multivariée, est étalonné, vérifié, ajusté et/ou entraîné à l'aide d'un spectromètre de laboratoire (31), notamment d'un spectromètre à émission atomique à plasma à couplage inductif, dans un laboratoire situé dans l'installation industrielle ou à distance de l'installation industrielle.

10. Système de mesure en ligne (100), le système de mesure en ligne (100) étant conçu pour mettre en œuvre le procédé selon au moins l'une des revendications précédentes, lequel système de mesure (100) comprend :
- un spectromètre ou spectrophotomètre (3) d'absorbance et/ou de réflectance en ligne dans le domaine ultraviolet ou ultraviolet-visible (3), destiné à déterminer en ligne un spectre d'absorbance et/ou de réflectance dans le domaine ultraviolet-visible ou ultraviolet,
- au moins un appareil de mesure en ligne (4, 5, 6, 7) distinct du spectromètre ou spectrophotomètre d'absorbance et/ou de réflectance dans le domaine de l'ultraviolet ou de l'ultraviolet-visible, ledit appareil de mesure en ligne étant conçu pour déterminer en ligne la concentration totale en sulfates métalliques (c_MeSO4) contenue dans la solution (1, 1a, 1b, ...), à l'aide d'au moins un procédé de mesure sensible à la concentration totale en sulfates métalliques, et
- une unité d'évaluation et/ou de calcul, laquelle est conçue pour déterminer la concentration au minimum en NiSO4, en CoSO et en MnSO4 (c_CoSO4, c_NiSO4, c_MnSO4) dans la solution (1, 1a, 1b, ...) .

11. Ensemble d'installation (200) destiné à la production d'une solution (1, 1a, 1b, ...) contenant des sulfates métalliques dans une installation industrielle, lequel ensemble comprend :
- une cuve (2) destinée à contenir la solution (1)
- un système de conduites comprenant des conduites (2a, 2b, 2c, ...) destinées à acheminer la solution (1, 1a, 1b, ...), les conduites (2a, 2b, 2c, ...) débouchant dans ou hors de la cuve (2), et
- au moins un premier système de mesure en ligne (100) selon la revendication 10, le premier système de mesure en ligne (100) étant installé dans le système de
conduites.

12. Ensemble d'installation (200) selon la revendication 11, comprenant :
- un premier, un deuxième, un troisième et, le cas échéant, un quatrième réservoir d'alimentation (12a, 12b, 12c, 12d), lesquels réservoirs sont destinés respectivement au premier, au deuxième, au troisième et, le cas échéant, au quatrième sulfate métallique,
- des conduites d'alimentation (13a, 13b, 13c, 13d) partant du premier, du deuxième, du troisième et, le cas échéant, du quatrième réservoir d'alimentation vers la cuve (2), pour produire la solution (1, 1a, 1b, ...),
l'ensemble d'installation (200) étant conçu de telle sorte que la concentration du premier, du deuxième, du troisième et, le cas échéant, du quatrième sulfate métallique (c_CoSO4, c_NiSO4, c_MnSO4, c_MxSO4) dans la solution (1, 1a, 1b, ...) soit déterminée à l'aide d'au moins le premier système de mesure en ligne (100), lors de son acheminement à partir de son réservoir d'alimentation respectif (12a, 12b, 12c, 12d) vers la cuve (2) pendant le mélange du sulfate métallique respectif à la solution (1, 1a, 1b, ...)
et l'ensemble d'installation (200) étant notamment conçu pour mélanger en ligne le premier, le deuxième, le troisième et, le cas échéant, le quatrième sulfate métallique les uns avec les autres, lors de leur acheminement à partir de leur réservoir d'alimentation respectif (12a, 12b, 12c, 12d) vers la cuve (2), de préférence par mélange statique.

13. Ensemble d'installation (200) selon au moins l'une des revendications 11 ou 12, comprenant un deuxième système de mesure en ligne (100b) tel que revendiqué dans la revendication 10, le deuxième système de mesure en ligne (100b) étant disposé dans une conduite de circuit (14), ladite conduite de circuit (14) partant d'une première sortie débouchant hors de la cuve (2) vers une entrée débouchant dans la cuve (2) et/ou
comprenant un troisième système de mesure en ligne (100c) tel que revendiqué dans la revendication 10, le troisième système de mesure en ligne (100c) étant disposé par rapport à une conduite d'alimentation (15), laquelle conduite d'alimentation (15) relie une deuxième sortie de la cuve à un cristalliseur de telle sorte que la concentration du premier, du deuxième, du troisième et, le cas échéant, du quatrième sulfate métallique (c_CoSO4, c_NiSO4, c_MnSO4 ; c_MxSO4) dans la solution (1, 1a, 1b, ...) est déterminée au cours de son trajet entre la cuve (2) et le cristalliseur.

14. Ensemble d'installation (200) selon au moins l'une des revendications 11 à 13,
pour lequel l'ensemble d'installation (200) est conçu pour réguler le débit entrant provenant des conduites d'alimentation (13a, 13b, 13c, 13d) vers la cuve (2), en fonction de la concentration du premier sulfate métallique, du deuxième sulfate métallique, du troisième sulfate métallique et/ou, le cas échéant, du quatrième sulfate métallique (c_CoSO4, c_NiSO4, c_MnSO4, c_MxSO4), déterminée en ligne à l'aide du premier système de mesure en ligne (100), du deuxième système de mesure en ligne (100b) et/ou du troisième système de mesure en ligne (100c).

15. Utilisation de l'ensemble d'installation (200) selon l'une des revendications 11 à 14, dans une conduite d'alimentation (15), lequel ensemble est destiné à un cristalliseur pour la cristallisation de matériaux de cathode pour batteries lithium-ion.
